(19) Europäisches Patentamt | European Patent Office | Office européen des brevets

(11) **EP 4 049 021 B1**

(12) **EUROPEAN PATENT SPECIFICATION**

(45) Date of publication and mention
of the grant of the patent:
**29.10.2025  Bulletin 2025/44**

(21) Application number: **20842701.3**

(22) Date of filing: **18.12.2020**

(51) International Patent Classification (IPC):
**G01N 30/04** *(2006.01)*     **G01N 30/86** *(2006.01)*
**A61K 36/09** *(2006.01)*     **B01D 15/36** *(2006.01)*
**B01D 15/12** *(2006.01)*

(52) Cooperative Patent Classification (CPC):
**B01D 15/363; A61K 36/09; B01D 15/12;**
G01N 30/8606; G01N 2030/884

(86) International application number:
**PCT/EP2020/087249**

(87) International publication number:
**WO 2021/123327 (24.06.2021 Gazette 2021/25)**

(54) **ANALYTICAL METHOD FOR ICELAND MOSS**

ANALYTISCHES VERFAHREN FÜR ISLAND-MOOS

PROCÉDÉ ANALYTIQUE DE MOUSSE D'ISLANDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.12.2019  SI 201900253**

(43) Date of publication of application:
**31.08.2022  Bulletin 2022/35**

(73) Proprietor: **KRKA, d.d., Novo mesto
8501 Novo mesto (SI)**

(72) Inventors:
• **MRZLIKAR, Miha
1360 Vrhnika (SI)**
• **KOVACIC, Simon
1000 Ljubljana (SI)**

(74) Representative: **Prüfer & Partner mbB
Patentanwälte · Rechtsanwälte
Sohnckestraße 12
81479 München (DE)**

(56) References cited:
• **GUDJONSDOTTIR G A ET AL: "Quantitative
determination of protolichesterinic- and
fumarprotocetraric acids in Cetraria islandica by
high-performance liquid chromatography",
JOURNAL OF CHROMATOGRAPHY A,
ELSEVIER, AMSTERDAM, NL, vol. 757, no. 1-2, 3
January 1997 (1997-01-03), pages 303 - 306,
XP004740004, ISSN: 0021-9673, DOI: 10.1016/
S0021-9673(96)00670-X**
• **WOOD PETER J. ET AL: "Structural studies of (1-
>3),(1->4)-beta -d-glucans by 13C-nuclear
magnetic resonance spectroscopy and by rapid
analysis of cellulose-like regions using high-
performance anion-exchange chromatography
of oligosaccharides released by lichenase",
CEREAL CHEMISTRY, vol. 71, no. 3, 1 January
1994 (1994-01-01), pages 301 - 307,
XP055789394, Retrieved from the Internet
<URL:https://www.cerealsgrains.org/
publications/cc/backissues/1994/Documents/
71_301.pdf> [retrieved on 20210323]**
• **INGOLFSDOTTIR K ET AL: "IMMUNOLOGICALLY
ACTIVE POLYSACCHARIDE FROM CETRARIA
ISLANDICA", PLANTA MEDICA, THIEME
VERLAG, DE, vol. 60, 1 January 1994
(1994-01-01), pages 527 - 531, XP002038400,
ISSN: 0032-0943, DOI: 10.1055/S-2006-959564**

EP 4 049 021 B1

- INGÓLFSDÓTTIR K. ET AL: "Immunomodulating polysaccharides from aqueous extracts of Cetraria islandica (Iceland moss)", PHYTOMEDICINE, vol. 5, no. 5, 1 October 1998 (1998-10-01), AMSTERDAM, NL, pages 333 - 339, XP055789289, ISSN: 0944-7113, DOI: 10.1016/S0944-7113(98)80014-7
- VICENTE C ET AL: "High-performance liquid chromatographic determination of sugars and polyols in extracts of lichens and sugarcane juice", JOURNAL OF CHROMATOGRAPHY A, ELSEVIER, AMSTERDAM, NL, vol. 553, 16 August 1991 (1991-08-16), pages 271 - 283, XP026759700, ISSN: 0021-9673, [retrieved on 19910816], DOI: 10.1016/S0021-9673(01)88498-3
- CASEIRO ET AL: "Determination of saccharides in atmospheric aerosol using anion-exchange high-performance liquid chromatography and pulsed-amperometric detection", JOURNAL OF CHROMATOGRAPHY A, ELSEVIER, AMSTERDAM, NL, vol. 1171, no. 1-2, 19 October 2007 (2007-10-19), pages 37 - 45, XP022307105, ISSN: 0021-9673, DOI: 10.1016/J.CHROMA.2007.09.038
- ETCHISON J R ET AL: "Carbohydrate composition of the membrane glycoprotein of vesicular stomatitis virus", VIROLOGY, ELSEVIER, AMSTERDAM, NL, vol. 60, no. 1, 1 July 1974 (1974-07-01), pages 217 - 229, XP023289902, ISSN: 0042-6822, [retrieved on 19740701], DOI: 10.1016/0042-6822(74)90379-1

**Description**

FIELD OF THE INVENTION

The present invention relates to an analytical method for quantitative determination of an Iceland moss herbal preparation in a herbal product.

BACKGROUND OF THE INVENTION

Iceland moss (Lichen islandicus) is the common name for the thallus of the lichen *Cetraria islandica* (L.) Archarius s.l.; *C. islandica* is a fruticose, terricolous lichen from the Parmeliaceae family growing in arctic and subarctic areas, in northern and eastern Europe, Siberia and North America and in middle ranged and alpine mountains in Germany, North Italy, Czech Republic, Slovakia, Poland and Russia. Iceland moss contains green alga *Trebouxia,* which can also be found in 75 % lichens. In lichens at least three species of *Trebouxia* exist - *Trebouxia decolorans, Trebouxia erici* and *Trebouxia glomerata.* However, their role in the lichens' carbohydrate metabolism may be similar and may be applied to many if not all of the 10.000 species.

Green algae produce genus-specific sugar alcohols - namely ribitol, sorbitol or erythritol. Ribitol (also known as adonitol and adonite) is a storage pentose alcohol synthesized during photosynthesis via xylulose-5-phosphate and pentose phosphate pathways by *Coccomyxa, Trentepohlia* and *Trebouxia* genus. Carbon in *Trebouxia* alga is mainly fixed into ribitol and to lower extent also into sucrose and other carbohydrate compounds. After synthesis in alga, only ribitol is moved to fungus, where it is metabolized into ribitol enantiomer called arabitol (also known as arabinitol) and in smaller amount also in mannitol. Polyols in lichens are osmoregulators, protein stabilizers, serve as carbon depo under stress conditions.

Iceland moss can be used as herbal tea to be drunk or in a form of a herbal preparation that is incorporated into a herbal product to be taken by mouth or applied to the lining of the mouth. Based on its long-standing and traditional use Iceland moss preparations can be used as a demulcent (soothing agent) for treating mouth and throat irritation and associated dry cough, as well as to treat temporary loss of appetite.

The Non-Patent Literature of G.A. Gudjönsdöttir and K. Ingölfsdöttir, Journal of Chromatography A, 757 (1997) 303-306, and titled "Quantitative determination of protolichesterinic- and fumarprotocetraric acids in *Cetraria islandica* by high-performance liquid chromatography", describes a quantitative reversed-phase HPLC method for the analysis of protolichesterinic- and fumarprotocetraric acids in *Cetraria islandica* (L.) Ach.. The compounds were extracted from dry plant material with acetone. HPLC analysis was performed using a LiChrosorb RP-8 column, isocratic elution and UV detection. The method was applied to the quantification of protolichesterinic- and fumarprotocetraric acids in plant material collected in various locations within Iceland. Results showed protolichesterinic acid content of *Cetraria islandica* to vary between 0.1-0.5% dry weight and fumarprotocetraric acid content to vary between 2.6-11.5% depending on collection site.

Further, the Non-Patent Literature of C. Vicente, Journal of Chromatography, 553 (1991) 271-283, and titled "High-performance liquid chromatographic determination of sugars and polyols in extracts of lichens and sugarcane juice" two lichens analyzed are of species *Evernia prunastri* and *Himantormia lugubris.* Ribitol is used as internal standard solely for method recovery correction, because it has similar chemical properties as other selected polyols (analytes).

SUMMARY OF THE INVENTION

In accordance with regulations, quality of herbal medicinal products should be guaranteed and demonstrated according to the existing requirements. Due to a specific and complex nature of herbal products, markers can provide an option to ensure and demonstrate adequate and consistent quality of these products. According to the Committee on Herbal Medicinal Products assessment report (https://www.ema.europa.eu/documents/herbal-report/final-assessment-report-cetraria-islandica-l-acharius-sl-thallus_en.pdf) Iceland moss herbal substance has no constituents with known therapeutic activity or active markers. It consists of the following groups of constituents: polysaccharides, lichen acids, minerals and other constituents. Concentration of these constituents or groups of them in the herbal preparation and herbal product can be very low; they can be unstable, hard to separate, interact with excipients and therefore unsuitable for quality control and present a great challenge for quantitative determination according to the current scientific practice.

The inventors of the present invention have surprisingly found that ribitol and arabitol, which are two of many substances in Iceland moss, can be used for analytical purpose as appropriate analytical markers for quantitative determination and also identification of Iceland moss herbal preparation in the herbal product. Therefore, ribitol or arabitol or both can be used for quantitative and qualitative purpose. They provide an important analytical tool to link the herbal preparation to the herbal product. Ribitol and arabitol serve as means of calculating the quantity of active substance (herbal preparation) in the finished product (herbal product) with a good correlation between the ribitol and arabitol quantity in the herbal preparation and the ribitol and arabitol quantity in the herbal product.

Accordingly, the present invention provides an analytical method for quantitative determination of an Iceland moss herbal preparation in a herbal product, wherein ribitol and/or arabitol is used as an analytical marker, wherein the analytical method is high-performance anion exchange chromatography. This analytical method can be used in a process for preparation of the herbal product.

The present invention further provides a process for producing a herbal product obtained from a herbal preparation, wherein during the production of the herbal product the quantity of the herbal preparation in the herbal product is determined by the aforementioned analytical method.

BRIEF DESCRIPTION OF THE DRAWINGS

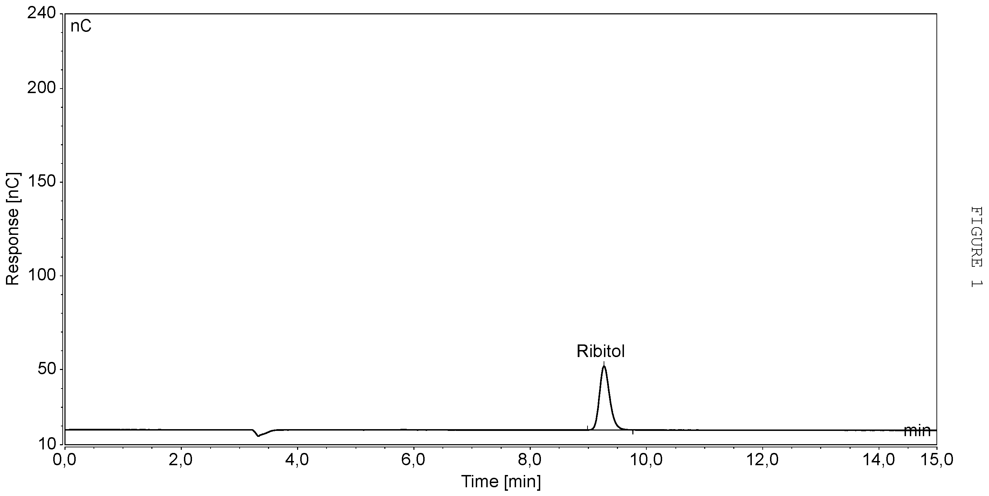

Figure 1 depicts chromatogram of the ribitol standard solution.

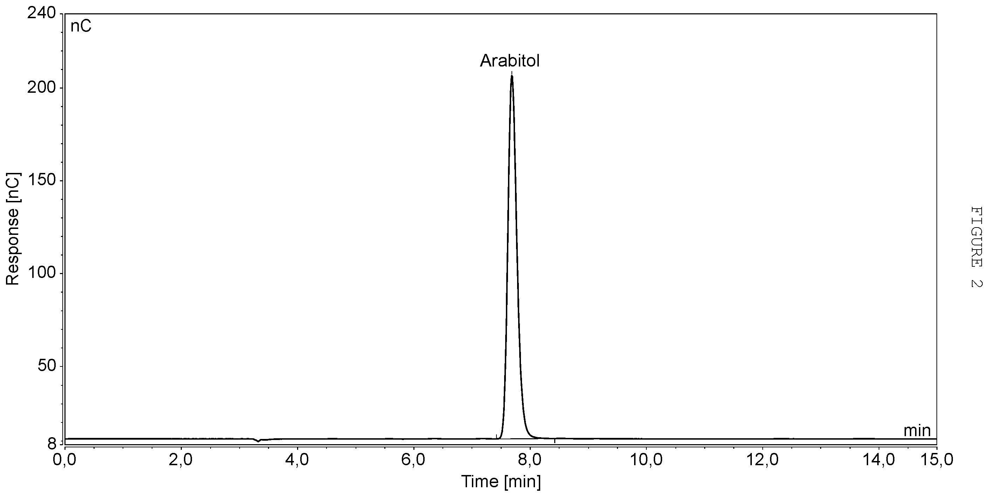

Figure 2 depicts chromatogram of the arabitol standard solution.

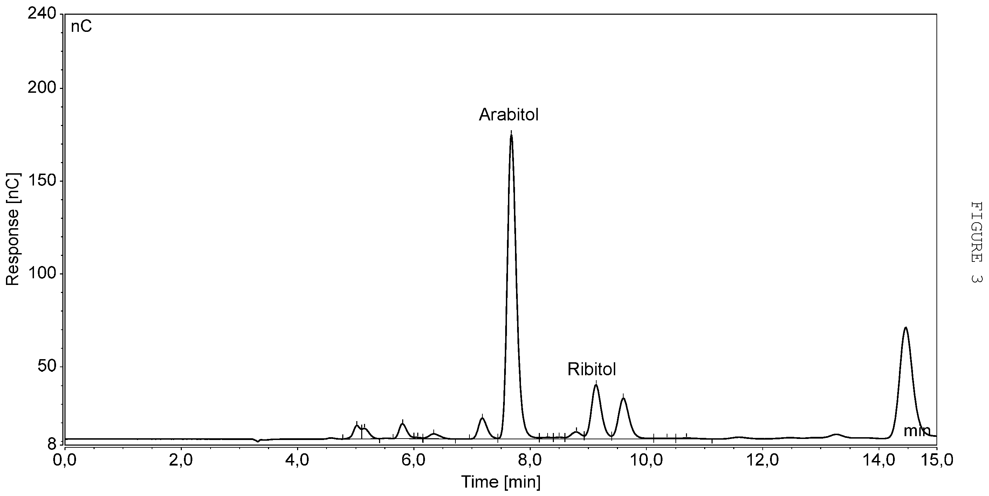

Figure 3 depicts chromatogram of the Iceland moss herbal preparation sample solution.

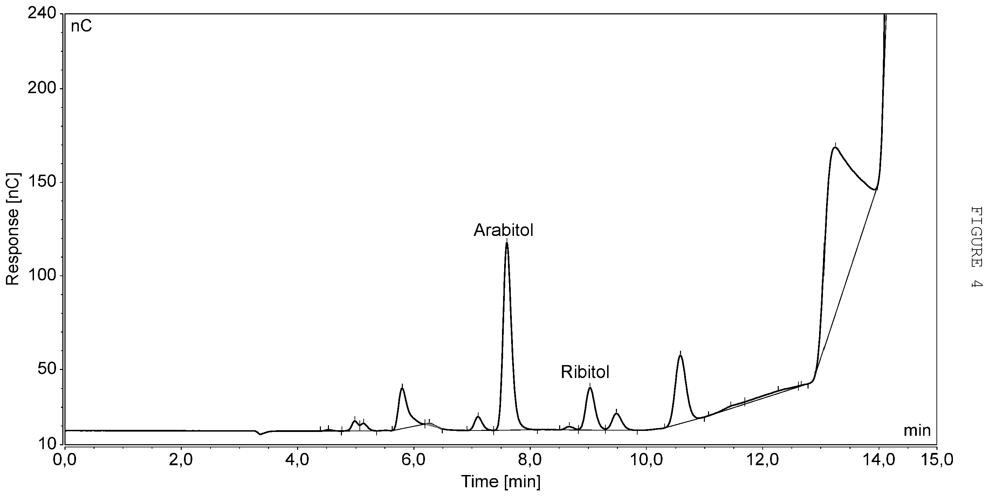

Figure 4 depicts chromatogram of the Iceland moss syrup sample solution (Example 2, composition A).

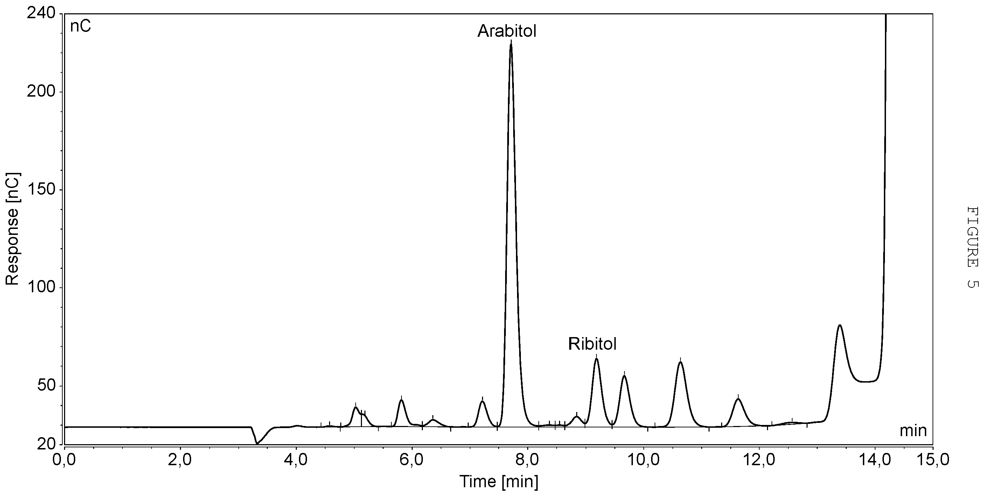

Figure 5 depicts chromatogram of the Iceland moss sugar based lozenge sample solution (Example 3, composition F).

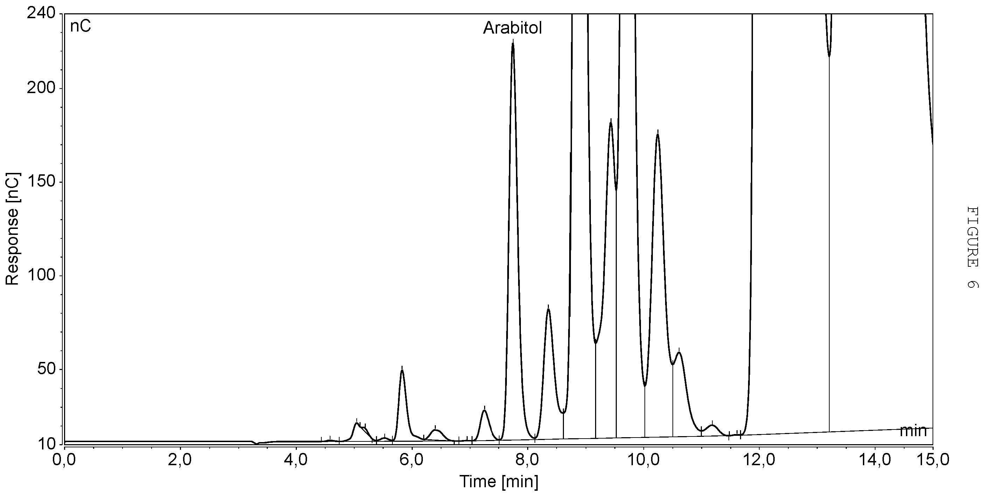

Figure 6 depicts chromatogram of the Iceland moss sugar-free lozenge sample solution (Example 3, composition C).

DETAILED DESCRIPTION OF THE INVENTION

The first aspect of the present invention is the analytical method for quantitative determination of an Iceland moss herbal preparation in a herbal product, wherein ribitol and/or arabitol is used as an analytical marker as specified above.

The term "quantitative determination" according to the present invention refers to determination of the absolute or relative abundance of the herbal preparation in the herbal product, which can be expressed in terms of concentration or mass fraction.

The term "herbal preparation" according to the present invention means any preparation obtained by subjecting an Iceland moss herbal substance to treatments such as extraction, distillation, expression, fractionation, purification, concentration or fermentation. These include comminuted or powdered herbal substance, tinctures, extracts, essential oils, expressed juices and processed exudates. Preferably, the herbal preparation according to the present invention is an extract, more preferably a water and/or water-ethanol extract and most preferably the herbal preparation is a water extract.

The term "herbal product" according to the present invention means any product comprising the Iceland moss herbal substance or Iceland moss herbal preparation. The herbal product can be presented in various dosage forms such as decoctions, herbal powders, alcoholic beverages, capsules, tablets, lozenges, syrups, ointments and creams. Preferably, the herbal product is an oral dosage form. More preferably, the herbal product according to the present invention is a syrup or a solid oral dosage form. Even more preferably, the solid oral dosage form is a tablet, capsule or a lozenge. Most preferably, the herbal product is a syrup or a lozenge.

The term "herbal substance" according to the present invention means whole, fragmented or cut Iceland moss in an unprocessed, usually dried form but sometimes fresh.

Markers are chemically defined constituents or groups of constituents of a herbal substance, a herbal preparation or a herbal product which are of interest for control purposes independent of whether they have any therapeutic activity. Markers serve to calculate the quantity of herbal substance(s) or herbal preparation(s) in the herbal product if the markers have been quantitatively determined in the herbal substance or herbal preparation. There are two categories of markers, active and analytical. Active markers are constituents or groups of constituents which are generally accepted to contribute to the therapeutic activity. Analytical markers are constituents or groups of constituents that serve for analytical purposes.

The analytical method of the present invention is a high-performance anion exchange chromatography (HPAE).

A stationary phase used in the HPAE chromatography according to the present invention can be anion exchange column, preferably the stationary phase is a strong anion exchange column, and the most preferred stationary phase is a high-capacity anion exchanger column based on a styrene-divinylbenzene copolymer.

Carbohydrates with a weakly acidic nature, such as sugar alcohols ribitol and arabitol, are selectively separated on a strong anion-exchange stationary phase. Due to use of a strong anion-exchange stationary phase, neutral and cationic compounds elute in, or close to the void volume and therefore do not interfere with the analytes of our interest. Following the separation of ribitol and/or arabitol from other constituents, such as carbohydrates, detection takes place on an appropriate detection system.

A mobile phase used in the liquid chromatography according to the present invention can be organic or inorganic. Preferably, the mobile phase is water or an aqueous solution. More preferably, the mobile phase is an alkaline aqueous solution. The aqueous solution can comprise acetate salts. Even more preferably, the mobile phase is an aqueous alkali hydroxide solution. The most preferable mobile phase is aqueous sodium hydroxide solution.

The analytical method according to the present invention preferably comprises the following steps:

(1) preparing a ribitol and/or arabitol standard solution;
(2) preparing a herbal preparation sample solution and/or herbal product sample solution;
(3) injecting separately into a chromatographic system the ribitol and/or arabitol standard solution, herbal preparation sample solution and herbal product sample solution;
(4) integrating ribitol and/or arabitol peaks; and
(5) determining the quantity of Iceland moss herbal preparation in the herbal product.

A detector used in the liquid chromatography according to the present invention can be a refractive index detector or a pulsed amperometric detector (PAD). The most preferable detector is the pulsed amperometric detector.

The most preferable analytical method according to the present invention is high-performance anion exchange chromatography coupled with the pulsed amperometric detector (HPAE-PAD).

In a herbal product the quantity of a herbal preparation in the herbal product may be determined by the analytical method of the present invention.

A further aspect of the present invention is a process for producing a herbal product obtained from a herbal preparation, wherein during the production of the herbal product the quantity of the herbal preparation in the herbal product is determined by the analytical method of the present invention. The analytical method using ribitol and/or arabitol as an analytical marker can be applied at any desired time during the entire herbal product production for quantitative determination of the Iceland moss herbal preparation in the herbal product.

The analytical method according to the present invention may be used in a process for preparation of the herbal product.

According to the present disclosure, ribitol and/or arabitol may be used as an analytical marker for an Iceland moss herbal preparation and/or a herbal product, notably as an analytical marker for quantitative determination of an Iceland moss herbal preparation in a herbal product. Ribitol or arabitol or both can be used as analytical markers - depending on the excipients in the herbal product and their possible interferences with ribitol or arabitol determination.

In the Examples, detection is achieved using pulsed amperometric detection (PAD). Pulsed amperometric detection is very sensitive, selective and specific means of determination. It only detects compounds containing oxidizable functional groups at the detection voltage employed that is specific for a class of compounds of interest and does not require any derivatization before or after separation step. Using PAD, analytes of interest (ribitol and/or arabitol) are detected by measuring the electrical current generated by their oxidation at the surface of a gold electrode. PAD detection usually employs a repeating sequence of a few potentials. In the Examples provided herein below the sequence consists of three potentials. Following the oxidation step from which only a portion of pulse is integrated and recorded, a cleaning step is performed where products of the oxidation reaction, which can have a negative effect on the electrode surface, are desorbed. This is accomplished by raising the potential to oxidize the electrode surface. During this process, a layer of gold oxide is formed and carbohydrate oxidation products are desorbed from the electrode. In the third and last step, the electrode surface is reduced back to gold by lowering the electrode potential.

EXAMPLES

Example 1: Determination of ribitol and/or arabitol content in the commercially available Iceland moss herbal preparation in the form of a soft water extract

| Composition of the extract | DER (drug extract ratio) |
| --- | --- |
| Iceland moss (whole or cut, dried thallus of *Cetraria islandica* (L.) Archarius s.l.) water extract | 16-18:1 |

Chromatographic conditions: column Metrosep Carb 2 (column dimensions: 250 mm × 4.0 mm, particle size: 5.0 μm); pre-column Metrosep Carb 2 Guard/4.0 (column dimensions: 5 mm × 4.0 mm, particle size: 5.0 μm). Other equivalent column and/or pre-column can also be used if demonstrated that it is fit for purpose. Column temperature: 40.0 °C, sample/standard solution temperature: room temperature. Mobile phase: gradient elution. Mobile phase A: 180 mM NaOH, mobile phase B: 700 mM NaOH. Gradient program: first step - separation: 0-15 min: 100 % A, 0 % B; second step - column clean-up: 16-35 min: 0 % A, 100 % B, third step - equilibration: 36-100 min: 100 % A, 0 % B. Flow rate 0.5 mL/min.

Note: The length of column clean-up step can be prolonged in case of delayed peaks. The length of equilibration step can be adjusted (prolonged or shortened) in order to ensure suitable chromatographic system.

Detection: pulsed amperometric detection. Working electrode: Au, reference electrode: Ag/AgCl. Pulse sequence:

| Time [s] | Potential [V] | Integration |
|---|---|---|
| 0.00 | 0.1 | OFF |
| 0.20 | 0.1 | ON |
| 0.40 | 0.1 | OFF |
| 0.41 | -2.0 | OFF |
| 0.42 | -2.0 | OFF |
| 0.43 | 0.6 | OFF |
| 0.44 | -0.1 | OFF |
| 0.50 | -0.1 | OFF |

Note: throughout the whole analytical procedure purified water for chromatography is used. The below described analytical procedures for herbal preparation and herbal products are based on external calibration method, however the sample solutions may also be prepared by other means of calibration - such as standard addition calibration method, but calculations have to be adjusted accordingly.

(1) Ribitol and/or arabitol standard solution is prepared in water at concentration of about 0.5 mg/L of ribitol and about 3.0 mg/L of arabitol. Standard solution(s) is/are prepared in two replicates.

(2) Sample solution is prepared by accurately weighing about 500 mg of the Iceland moss herbal preparation into an appropriate Erlenmeyer flask and adding about 100 mL of water. The sample is completely dissolved using ultra sound for about 15 to 30 min. Meanwhile the mixture is shaken several times. Solution is transferred into a 200 mL volumetric flask and the Erlenmeyer flask is washed several times with water in order to achieve a quantitative transfer of the sample solution. Volumetric flask is filled to volume with water and mixed well (Sample solution 1 = SaS1). 1.0 mL of SaS1 is transferred into a 50 mL volumetric flask and the SaS1 aliquot is weighed. The flask is filled to volume with water and the prepared solution is weighed (Sample solution 2 = SaS2). SaS2 concentration is about 50 mg/L.

(3) First solvent (water, purified for chromatography) is injected into equilibrated chromatographic system. Then ribitol and/or arabitol standard solution is injected into equilibrated chromatographic system three times in succession and using ribitol and/or arabitol peaks from chromatograms obtained relative standard deviation (RSD) is calculated. RSD should not exceed 3 %.

(4) Second ribitol and/or arabitol standard solution is injected into equilibrated chromatographic system once and its/their area (or content) on the chromatogram should not differ from the average peak area (or content) of the first ribitol and/or arabitol standard solution for more than 1.5 %.

(5) Sample solution is injected into equilibrated chromatographic system.

(6) Ribitol and/or arabitol content (in %) in the Iceland moss herbal preparation is calculated using the following equation:

$$\frac{A_{\text{SaS}} \times C_{SS} \times W_{\text{SaS2}} \times 200}{A_{SS} \times W_{SaS1,aliquot} \times W \times 1000} \times 100$$

where:

$A_{\text{SaS2}}$ = peak area of ribitol on the chromatogram of the sample solution
$A_{SS}$ = average peak area of ribitol on the chromatograms of the standard solution
$C_{SS}$ = concentration of ribitol in the standard solution [mg/L]
200 = volume of the sample solution 1 [mL]
W = sample weight [mg]
$W_{\text{SaS2}}$ = sample solution 2 weight [mg]
$W_{\text{SaS1,aliquot}}$ = weight of a sample solution 1 aliquot [mg]

Arabitol content (in %) can be calculated the same way as ribitol content, only peak areas of aribitol and concentration of arabitol in the standard solution is taken into account instead.

Quantitative determination of ribitol in Iceland moss herbal preparation in the form of a soft extract yielded a result of 0.8094 %.

$$\frac{A_{SaS} \times C_{SS} \times W_{SaS2} \times 200}{A_{SS} \times W_{SaS1,aliquot} \times W \times 1000} \times 100$$

$$= \frac{5.972926 \text{ nC} \cdot \text{min} \times 0.496980 \frac{\text{mg}}{\text{L}} \times 49914.7 \text{ mg} \times 200 \text{ mL}}{7.256233 \text{ nC} \cdot \text{min} \times 995.4 \text{ mg} \times 506.9 \, mg \times 1000} \times 100 = 0.8094 \%$$

Quantitative determination of arabitol in Iceland moss herbal preparation in the form of a soft extract yielded a result of 5.0545%.

$$\frac{A_{SaS} \times C_{SS} \times W_{SaS2} \times 200}{A_{SS} \times W_{SaS1,aliquot} \times W \times 1000} \times 100$$

$$= \frac{30.196043 \text{ nC} \cdot \text{min} \times 2.993760 \frac{\text{mg}}{\text{L}} \times 49914.7 \text{ mg} \times 200 \text{ mL}}{35.385999 \text{ nC} \cdot \text{min} \times 995.4 \text{ mg} \times 506.9 \, mg \times 1000} \times 100 = 5.0545\%$$

Example 2: Quantitative determination of the Iceland moss herbal preparation in the herbal product in the form of a syrup

Composition of the syrup

Compositions A-C

| Ingredient | Quantity [mg/mL] | | |
|---|---|---|---|
| | A | B | C |
| Iceland moss soft extract | 6.0 | 6.0 | 6.0 |
| Fructose | 532 | 532 | 532 |
| Xanthan gum | 1.0 | 2.0 | 2.0 |
| Sodium benzoate | 2.0 | 2.0 | 1.5 |
| Citric acid monohydrate | 0.4 | 0.4 | 0.4 |
| Flavour lemon | 2.0 | 2.0 | 2.0 |
| Water, purified | ad 1 ml | ad 1 ml | ad 1 ml |

Preparation process for the syrup

Sodium benzoate is dissolved in purified water and the solution is heated to 50 - 70 °C. At this temperature Iceland moss soft extract is added and homogenously dispersed. Dispersion is then heated to 90 - 95 °C, maintained at this temperature for at least 20 minutes, and then cooled to 50 - 70 °C at which temperature fructose is added and mixed until dissolved. After the addition of fructose, the dispersion is cooled to room temperature. Xanthan gum, citric acid and flavouring agent are added at room temperature and mixed until dissolved. Syrup can be filtered to eliminate particulate impurities, before being transferred into suitable glass bottles.

Analytical method

(1) Chromatographic conditions are the same as stated in Example 1.

(2) Placebo solution (blank solution) is prepared by weighing about 500 mg of a placebo (syrup without the herbal preparation)into a 50 mL volumetric flask. The placebo is dissolved in water, the flask is filled with the same solvent to volume and mixed well.

(3) Ribitol and/or arabitol stock standard solution is prepared in water. Working standard solution is prepared by weighing about 500 mg of a placebo into a 50 mL volumetric flask. Then an appropriate volume of the stock standard solution of ribitol and/or arabitol is added, the mixture is dissolved in water and the flask is filled with the same solvent to volume and mixed well, making the solution which contains about 0.5 mg/L of ribitol and/or about 3.0 mg/L of arabitol and about 10,000 mg/L of placebo. Standard solution(s) is/are prepared in two replicates.

(4) Sample solution is prepared by accurately weighing about 500 mg of the herbal product (syrup) into a 50 mL volumetric flask. The syrup is dissolved in water, the flask filled with the same solvent to volume and mixed well. Sample solution concentration is about 10,000 mg/L.

(5) First placebo solution is injected into equilibrated chromatographic system. Then ribitol and/or arabitol standard solution is injected into equilibrated three times in succession and using ribitol and/or arabitol peaks from chromatograms obtained relative standard deviation (RSD) is calculated. RSD should not exceed 3 %.

(6) Second ribitol and/or arabitol standard solution is injected into equilibrated chromatographic system once and its area (or content) on the chromatogram should not differ from the average peak area (or content) of the first ribitol and/or arabitol standard solution for more than 1.5 %.

(7) Sample solution is injected into equilibrated chromatographic system.

(8) Ribitol and/or arabitol content (in ppm) in the herbal product (syrup) is calculated using the following equation:

$$\frac{A_{Sas} \times C_{SS} \times 50 \times 1 \cdot 10^6}{A_{SS} \times W \times 1000}$$

The content of the Iceland moss herbal preparation (expressed as a percentage of the stated amount) in the herbal product (syrup) is calculated using the following equation:

$$\frac{\dfrac{A_{Sas} \times C_{SS} \times 50 \times \rho}{A_{SS} \times W}}{SA \times \dfrac{C_{HP}}{100}} \times 100$$

where:

$A_{Sas}$ = peak area of ribitol on the chromatogram of the herbal product sample solution

$A_{SS}$ = average peak area of ribitol on the chromatograms of the ribitol standard solutions

$C_{SS}$ = concentration of ribitol in the standard solution [mg/L]

50 = volume of the stock sample solution [mL]

W = sample weight [mg]

$\rho$ = herbal product density [g/mL]

SA = stated amount of Iceland moss herbal preparation in the herbal product [mg/mL]

$C_{HP}$ = concentration of ribitol in the entering batch of Iceland moss herbal preparation [%]

Arabitol content can be calculated the same way as ribitol content, only peak areas of aribitol and concentration of arabitol in the standard solution is taken into account instead.

Excipients in the composition A do not interfere neither with ribitol neither with arabitol peaks. Therefore, either or both analytical markers can be used for quantitative determination and also identification of Iceland moss herbal preparation in the herbal product.

Quantitative determination of ribitol in herbal product in the form of a syrup prepared from Iceland moss yielded a result of 41.1 ppm (according to the first equation), while determination of arabitol yielded a result of 259.1 ppm. Taking into calculation quantity of ribitol in the entering batch of Iceland moss herbal preparation (soft extract), this equals to 101.6 % (according to the second equation) or 102.5 % when taking quantity of arabitol in the entering batch into calculation.

Example of calculation of Iceland moss extract content for composition A (analytical marker: ribitol) in syrup:

$$\frac{A_{SaS} \times C_{SS} \times 50 \times 1 \cdot 10^6}{A_{SS} \times W \times 1000} = \frac{5.776557 \, nC \cdot min \times 0.498960 \frac{mg}{L} \times 50 \, mL \times 1 \cdot 10^6}{6.597629 \, nC \cdot min \times 531.4 \, mg \times 1000}$$

$$= 41.1 \, ppm$$

$$\frac{\dfrac{A_{SaS} \times C_{SS} \times 50 \times \rho}{A_{SS} \times W}}{SA \times \dfrac{C_{HP}}{100}} \times 100$$

$$= \frac{\dfrac{5.776557 \, nC \cdot min \times 0.498960 \frac{mg}{L} \times 50 \, mL \times 1.19995 \, g/mL}{6.597629 \, nC \cdot min \times 531.4 \, mg}}{6 \, mg/mL \times \dfrac{0.809382 \, \%}{100}} \times 100$$

$$= 101.6 \, \%$$

Example of calculation of Iceland moss extract content for composition A (analytical marker: arabitol) in syrup:

$$\frac{A_{SaS} \times C_{SS} \times 50 \times 1 \cdot 10^6}{A_{SS} \times W \times 1000} = \frac{15.759129 \, nC \cdot min \times 2.993760 \frac{mg}{L} \times 50 \, mL \times 1 \cdot 10^6}{18.076994 \, nC \cdot min \times 503.7 \, mg \times 1000}$$

$$= 259.1 \, ppm$$

$$\frac{\dfrac{A_{SaS} \times C_{SS} \times 50 \times \rho}{A_{SS} \times W}}{SA \times \dfrac{C_{HP}}{100}} \times 100$$

$$= \frac{\dfrac{15.759129 \, nC \cdot min \times 2.993760 \frac{mg}{L} \times 50 \, mL \times 1.19995 \, g/mL}{18.076994 \, nC \cdot min \times 503.7 \, mg}}{6 \, mg/mL \times \dfrac{5.054452 \, \%}{100}} \times 100$$

$$= 102.5 \, \%$$

Using ribitol or arabitol as analytical markers for determination of Iceland moss extract content in the herbal product yields comparable results, well within the possible error of the analytical method.

Example 3: Quantitative determination of the Iceland moss herbal preparation in the herbal product in the form of a lozenge

Compositions A-H

| Ingredient | Quantity [mg] | | | | | | | |
|---|---|---|---|---|---|---|---|---|
| | A | B | C | D | E | F | G | H |
| Isomalt | 2433 | / | 2438 | / | 2438 | / | 2458 | / |

(continued)

| Ingredient | Quantity [mg] | | | | | | | |
|---|---|---|---|---|---|---|---|---|
| | A | B | C | D | E | F | G | H |
| Sucrose | / | 1185 | / | 1187 | / | 1187 | / | 1185 |
| Maize starch syrup (dry matter) | / | 1248 | / | 1251 | / | 1251 | / | 1248 1 |
| Iceland moos soft extract | 35.0 | 35.0 | 35.0 | 35.0 | 35.0 | 35.0 | 35.0 | 35.0 |
| Sucralose | 1.5 | 1.50 | 1.5 | 1.5 | 1.5 | 1.5 | 1.5 | 1.5 |
| Ascorbic acid | 25.0 | 25.0 | / | / | / | / | / | / |
| Malic acid | / | / | / | 20.0 | / | / | / | / |
| Citric acid | / | / | 20.0 | / | / | 20.0 | / | / |
| Tartaric acid | / | / | / | / | / | / | / | 25.0 |
| Lactic acid | / | / | / | / | 20.0 | / | / | / |
| Colour Carmine 52% (E120) | / | / | / | 0.5 | / | / | 0.5 | 0.5 |
| Colour Curcumine (E100) | 0.5 | 0.5 | 0.5 | / | 0.5 | 0.5 | / | / |
| Flavor pear | 5.0 | 5.0 | 5.0 | | 5.0 | 5.0 | / | / |
| Flavor wild strawberries | / | / | / | 5.0 | / | / | 5.0 | 5.0 |
| Flavor strawberries | / | / | / | / | / | / | / | / |

Compositions I-N

| Ingredient | Quantity [mg] | | | | | |
|---|---|---|---|---|---|---|
| | I | J | K | L | M | N |
| Isomalt | / | 2460 | / | 2458 | / | / |
| Sucrose | 1187 | / | 1198 | / | 1197 | 1187 |
| Maize starch syrup (dry matter) | 1251 | / | 1262 | / | 1261 | 1251 |
| Iceland moos soft extract | 35.0 | 35.0 | 35.0 | 35.0 | 35.0 | 35.0 |
| Sucralose | 1.5 | / | / | 1.5 | 1.5 | 1.5 |
| Ascorbic acid | / | / | / | / | / | / |
| Malic acid | / | / | / | / | / | / |
| Citric acid | / | / | / | / | / | 20.0 |
| Tartaric acid | / | / | / | / | / | / |
| Lactic acid | 20.0 | / | / | / | / | / |
| Colour Carmine 52% (E120) | / | / | / | 0.7 | 0.5 | 0.5 |
| Colour Curcumine (E100) | 0.5 | 0.5 | 0.5 | / | / | / |
| Flavor pear | 5.0 | 5.0 | 5.0 | / | / | / |
| Flavor wild strawberries | / | / | / | 5.0 | 5.0 | / |
| Flavor strawberries | / | / | / | / | / | 5.0 |

Preparation process of lozenges

Sugar based lozenges are manufactured in a special process on dedicated equipment. Sucrose, starch syrup and sucralose are dissolved in purified water and then cooked and vacuumed in a vacuum chamber to reduce moisture content. Iceland moos soft extract is added simultaneously by feeder. Organic acid, flavoring agents and colorants are dissolved in purified water or anhydrous ethanol and separately added by dosing pumps. Melted mixture is formed into lozenges in a lozenge former and lozenges are solidified in a cooling tunnel.

Sugar-free lozenges are manufactured in a special process on dedicated equipment. Isomalt and sucralose are dissolved in purified water and then cooked and vacuumed in a vacuum chamber to reduce moisture content. Iceland moos soft extract is added simultaneously by paste feeder. Organic acid, flavoring agents and colorants are dissolved in purified water or anhydrous ethanol and separately added by dosing pumps. Melted mixture is formed into lozenges in a lozenge former and lozenges are solidified in a cooling tunnel.

Compositions O-T (center filled lozenges)

| Ingredient | Quantity [mg] | | | | | |
|---|---|---|---|---|---|---|
| | O | P | Q | R | S | T |
| Isomalt | 2218 | 2218 | 2218 | 2223 | 2223 | 2223 |
| Center fill | 250.0 | 250.0 | 250.0 | 250.0 | 250.0 | 250.0 |
| | C1 | C2 | C3 | C4 | C5 | C6 |
| Sucralose | 1.5 | 1.5 | 1.5 | 1.0 | 1.0 | 1.0 |
| Malic acid | 25.0 | 25.0 | 25.0 | / | / | / |
| Tartaric acid | / | / | / | 20.5 | 20.5 | 20.5 |
| Colour Carmine 52% (E120) | / | / | / | 0.5 | 0.5 | 0.5 |
| Colour Curcumine (E100) | 0.5 | 0.5 | 0.5 | / | / | / |
| Flavor pear | 5.0 | 5.0 | 5.0 | / | / | / |
| Flavor wild strawberries | / | / | / | 5.0 | 5.0 | / |
| Flavor strawberries | / | / | / | / | / | 5.0 |

Compositions of center fills C1-C6:

| Ingredient | Quantity [mg] | | | | | |
|---|---|---|---|---|---|---|
| | C1 | C2 | C3 | C4 | C5 | C6 |
| Isomalt | 178.0 | 178.0 | 178.0 | 178.2 | 178.2 | 178.2 |
| Purified water | 35.3 | 35.3 | 35.3 | 35.3 | 35.3 | 35.3 |
| Iceland moos soft extract | 35.0 | 35.0 | 35.0 | 35.0 | 35.0 | 35.0 |
| Sucralose | 0.2 | 0.2 | 0.2 | / | / | / |
| Flavor pear | 1.5 | / | / | 1.5 | / | / |
| Flavor wild strawberries | / | 1.5 | / | / | 1.5 | / |
| Flavor strawberries | / | / | 1.5 | / | / | 1.5 |
| Total quantity of center fill | 250.0 | 250.0 | 250.0 | 250.0 | 250.0 | 250.0 |

Preparation process of sugar-free center fill lozenges

Isomalt based center filled lozenges are manufactured in a special process on dedicated equipment. Center fill is prepared by dissolving isomalt in purified water and heating it up to 110°C. Obtained isomalt syrup is cooled down to 80°C.

Iceland moos soft extract, sucralose and flavors are added to isomalt syrup during continuous mixing to obtain center fill mass. Center fill mass is continuously stirred and kept at approximately 65°C. For preparation of lozenges isomalt, organic acid and sucralose are dissolved in purified water and then cooked and vacuumed in a vacuum chamber to reduce moisture content. Flavoring agents and colorants are dissolved in purified water or anhydrous ethanol are added by dosing pumps and center fill is added separately by special pump. Lozenge mass is formed into center-filled lozenges in a lozenge former and lozenges are solidified in a cooling tunnel.

Analytical method

(1) Chromatographic conditions are the same as stated in Example 1.

(2) Ribitol and/or arabitol standard solution is prepared in water at concentration of about 0.5 mg/L of ribitol and about 3.0 of arabitol. Standard solution(s) is/are prepared in two replicates.

Note: If needed (to ensure suitable chromatographic system) standard solution of ribitol and/or arabitol can be prepared in placebo (lozenges without the herbal preparation) solution (about 4.000 mg/L of placebo in water).

(3) Sample solution is prepared by weighing at least 10 units of lozenges and determining the average weight. Then the lozenges are finely ground and homogenized in a laboratory mill. About 200 mg of the homogenized sample is accurately weighed into a 50 mL volumetric flask. Then about 25 mL of water are added and sample is dissolved using ultra sound for about 15 min to 30 min. Meanwhile the mixture is shaken several times. At the end the flask is filled to volume with water and mixed well. Sample solution concentration is about 4.000 mg/L.

Alternatively, a sample solution can be prepared according to the following procedure: 10 units of lozenges are weighed into an appropriate Erlenmeyer flask and adding about 300 mL of water. The sample is completely dissolved using ultra sound for about 15 to 30 min. Meanwhile the mixture is shaken several times. Solution is transferred into a 500 mL volumetric flask and the Erlenmeyer flask is washed several times with water in order to achieve a quantitative transfer of the sample solution. Volumetric flask is filled to volume with water and mixed well (Sample solution 1 = SaS1). 4.0 mL of SaS1 is transferred into a 50 mL volumetric flask and the SaS1 aliquot is weighed. The flask is filled to volume with water and the prepared solution is weighed (Sample solution 2 = SaS2). SaS2 concentration is about 4000 mg/L. (Note: The calculations of the content should be adjusted according to the sample preparation procedure used.)

(4) First solvent solution (water, purified for chromatography) is injected into equilibrated chromatographic system. Then ribitol and/or arabitol standard solution is injected into equilibrated chromatographic system three times in succession and using ribitol and/or arabitol peaks from chromatograms obtained relative standard deviation (RSD) is calculated. RSD should not exceed 3 %.

(5) Second ribitol and/or arabitol standard solution is injected into equilibrated chromatographic system once and its area on the chromatogram (or content) should not differ from the average peak area (or content) of the first ribitol and/or arabitol standard solution for more than 1.5 %.

(6) Sample solution is injected into equilibrated chromatographic system.

(7) Ribitol and/or arabitol content (in ppm) in the herbal product (lozenges) is calculated using the following equation:

$$\frac{A_{SaS} \times C_{SS} \times 50 \times 1 \cdot 10^6}{A_{SS} \times W \times 1000}$$

The content of the Iceland moss herbal preparation (expressed as a percentage of the stated amount) in the herbal product (lozenges) is calculated using the following equation:

$$\frac{\dfrac{A_{SaS} \times C_{SS} \times 50}{A_{SS} \times W \times 1000}}{\dfrac{SA}{AW} \times \dfrac{C_{HP}}{100}} \times 100$$

where:

$A_{SaS}$ = peak area of ribitol on the chromatogram of the sample solution

$A_{SS}$ = average peak area of ribitol on the chromatograms of the standard solution

$C_{SS}$ = concentration of ribitol in the standard solution [mg/L]

50 = volume of the stock sample solution [mL]

W = sample weight [mg]

SA = stated amount of Iceland moss herbal preparation in the herbal product in form of lozenges [mg]

AW - average weight of a lozenge [mg]

$C_{HP}$ = concentration of ribitol in the entering batch of Iceland moss herbal preparation [%]

Arabitol content can be calculated the same way as ribitol content, only peak areas of aribitol and concentration of arabitol in the standard solution is taken into account instead. Excipients of the herbal product in the form of sugar based lozenges with composition F interfere with arabitol peak, while excipients of the presented sugar-free composition C interfere with ribitol peak. Therefore, only one analytical marker of Iceland moss can be used for quantitative determination of Iceland moss herbal preparation in two of the presented examples of herbal products (C and F). For composition F (sugar based lozenges) this is ribitol and for composition C (sugar-free lozenges) arabitol.

Quantitative determination of ribitol in herbal product in the form of sugar based lozenges (composition F) prepared from Iceland moss yielded a result of 113.9 ppm (according to the first equation), while determination of arabitol in sugar-free lozenges (composition C) yielded a result of 734.2 ppm. Taking into calculation quantity of ribitol in the entering batch of Iceland moss herbal preparation (soft extract), this equals to 100.5 % (according to the second equation) for example F and 103.8 % when taking quantity of arabitol in the entering batch into calculation for composition C.

Example of calculation of Iceland moss extract content (analytical marker: ribitol) in sugar based lozenges (composition F):

$$\frac{A_{SaS} \times C_{SS} \times 50 \times 1 \cdot 10^6}{A_{SS} \times W \times 1000} = \frac{6.028414\,\text{nC}\cdot\text{min} \times 0.500940\,\frac{\text{mg}}{\text{L}} \times 50\,\text{mL} \times 1 \cdot 10^6}{6.590891\,\text{nC}\cdot\text{min} \times 201.2\,mg \times 1000}$$

$$= 113.9\,ppm$$

$$\frac{\dfrac{A_{SaS} \times C_{SS} \times 50}{A_{SS} \times W \times 1000}}{\dfrac{SA}{AW} \times \dfrac{C_{HP}}{100}} \times 100 = \frac{\dfrac{6.028414\,\text{nC}\cdot\text{min} \times 0.500940\,\frac{\text{mg}}{\text{L}} \times 50\,mL}{6.590891\,\text{nC}\cdot\text{min} \times 201.2\,mg \times 1000}}{\dfrac{35\,mg}{2500\,mg} \times \dfrac{0.809382\,\%}{100}} \times 100$$

$$= 100.5\,\%$$

Example of calculation of Iceland moss extract content (analytical marker: arabitol) in sugar free lozenges (composition C):

$$\frac{A_{SaS} \times C_{SS} \times 50 \times 1 \cdot 10^6}{A_{SS} \times W \times 1000} = \frac{36.199237 \cdot \text{min} \times 2.993760\,\frac{\text{mg}}{\text{L}} \times 50\,\text{mL} \times 1 \cdot 10^6}{36.681182\,\text{nC}\cdot\text{min} \times 201.2\,mg \times 1000}$$

$$= 734.2\,ppm$$

$$\frac{\dfrac{A_{SaS} \times C_{SS} \times 50}{A_{SS} \times W \times 1000}}{\dfrac{SA}{AW} \times \dfrac{C_{HP}}{100}} \times 100 = \frac{\dfrac{36.199237\,\text{nC}\cdot\text{min} \times 2.993760\,\frac{\text{mg}}{\text{L}} \times 50\,mL}{36.681182\,\text{nC}\cdot\text{min} \times 201.2\,mg \times 1000}}{\dfrac{35\,mg}{2500\,mg} \times \dfrac{5.054452\,\%}{100}} \times 100$$

$$= 103.8\,\%$$

## Claims

1.   An analytical method for quantitative determination of an Iceland moss herbal preparation in a herbal product,

wherein ribitol and/or arabitol is used as an analytical marker,
wherein the analytical method is high-performance anion exchange chromatography.

2. The analytical method according to claim 1, wherein a detector used in the high-performance anion exchange chromatography is a pulsed amperometric detector.

3. The analytical method according to claim 1, wherein a mobile phase used in the liquid chromatography is water or an aqueous solution.

4. The analytical method of claim 1, wherein the analytical method comprises the following steps:

a) preparing a ribitol and/or arabitol standard solution;
b) preparing a herbal preparation sample solution and/or herbal product sample solution;
c) injecting separately into a chromatographic system the ribitol and/or arabitol standard solution, herbal preparation sample solution and herbal product sample solution;
d) integrating ribitol and/or arabitol peaks; and
e) determining the quantity of Iceland moss herbal preparation in the herbal product.

5. The analytical method according to claim 1 used in a process for preparation of the herbal product.

6. The analytical method according to claim 1, wherein the herbal product is an oral dosage form.

7. The analytical method according to claim 6, wherein the oral dosage form is a syrup or a lozenge.

8. The analytical method according to claim 1, wherein the herbal preparation is an extract.

9. A process for producing a herbal product obtained from a herbal preparation, wherein during the production of the herbal product the quantity of the herbal preparation in the herbal product is determined by the analytical method of claim 1.


**Patentansprüche**

1. Analytisches Verfahren zur quantitativen Bestimmung einer Island-Moos-Pflanzenzubereitung in einem pflanzlichen Produkt,

wobei als analytischer Marker Ribitol und/oder Arabitol verwendet wird,
wobei das analytische Verfahren Hochleistungsanionenaustauschchromatographie ist.

2. Analytisches Verfahren gemäß Anspruch 1, wobei ein in der Hochleistungsanionenaustauschchromatographie verwendeter Detektor ein gepulster amperometrischer Detektor ist.

3. Analytisches Verfahren gemäß Anspruch 1, wobei eine in der Flüssigchromatographie verwendete mobile Phase Wasser oder eine wässrige Lösung ist.

4. Analytisches Verfahren gemäß Anspruch 1, wobei das analytische Verfahren die folgenden Schritte umfasst:

a) Herstellen einer Ribitol- und/oder Arabitol-Standardlösung;
b) Herstellen einer pflanzlichen Zubereitungsprobenlösung und/oder pflanzlichen Produktprobenlösung;
c) getrenntes Injizieren der Ribitol- und/oder Arabitol-Standardlösung, der pflanzlichen Zubereitungsprobenlösung und der pflanzlichen Produktprobenlösung in ein chromatographisches System;
d) Integrieren von Ribitol- und/oder Arabitol-Peaks; und
e) Bestimmen der Menge der Island-Moos-Pflanzenzubereitung in dem pflanzlichen Produkt.

5. Analytisches Verfahren gemäß Anspruch 1, das in einem Verfahren zur Herstellung des pflanzlichen Produkts verwendet wird.

6. Analytisches Verfahren gemäß Anspruch 1, wobei das pflanzliche Produkt eine orale Darreichungsform ist.

**7.** Analytisches Verfahren gemäß Anspruch 6, wobei die orale Darreichungsform ein Sirup oder eine Lutschtablette ist.

**8.** Analytisches Verfahren gemäß Anspruch 1, wobei die pflanzliche Zubereitung ein Extrakt ist.

**9.** Verfahren zur Herstellung eines pflanzlichen Produkts, das aus einer pflanzlichen Zubereitung erhalten wird, wobei während der Herstellung des pflanzlichen Produkts die Menge der pflanzlichen Zubereitung in dem pflanzlichen Produkt durch das analytische Verfahren gemäß Anspruch 1 bestimmt wird.

**Revendications**

**1.** Procédé analytique pour la détermination quantitative d'une préparation de plantes de mousse d'Islande dans un produit à base de plantes,

où du ribitol et/ou de l'arabitol est utilisé en tant que marqueur analytique,
où le procédé analytique est une chromatographie d'échange d'anions à haute performance.

**2.** Procédé analytique selon la revendication 1, où un détecteur utilisé dans la chromatographie d'échange d'anions à haute performance est un détecteur ampérométrique pulsé.

**3.** Procédé analytique selon la revendication 1, où une phase mobile utilisée dans la chromatographie liquide est de l'eau ou une solution aqueuse.

**4.** Procédé analytique selon la revendication 1, où le procédé analytique comprend les étapes suivantes :

a) préparation d'une solution étalon de ribitol et/ou d'arabitol ;
b) préparation d'une solution d'échantillon de préparation de plantes et/ou d'une solution d'échantillon de produit à base de plantes ;
c) injection séparée dans un système chromatographique de la solution étalon de ribitol et/ou d'arabitol, de la solution d'échantillon de préparation de plantes et de la solution d'échantillon de produit à base de plantes ;
d) intégration de pics de ribitol et/ou d'arabitol ; et
e) détermination de la quantité de préparation de plantes de mousse d'Islande dans le produit à base de plantes.

**5.** Procédé analytique selon la revendication 1 utilisé dans un procédé de préparation du produit à base de plantes.

**6.** Procédé analytique selon la revendication 1, où le produit à base de plantes est une forme posologique orale.

**7.** Procédé analytique selon la revendication 6, où la forme posologique orale est un sirop ou une pastille.

**8.** Procédé analytique selon la revendication 1, où la préparation de plantes est un extrait.

**9.** Procédé de production d'un produit à base de plantes obtenu à partir d'une préparation de plantes, où pendant la production du produit à base de plantes, la quantité de la préparation de plantes dans le produit à base de plantes est déterminée par le procédé analytique selon la revendication 1.

FIGURE 1

FIGURE 2

FIGURE 3

FIGURE 4

FIGURE 5

EP 4 049 021 B1

FIGURE 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **G.A. GUDJÖNSDÖTTIR** ; **K. INGÖLFSDÖTTIR**. *Journal of Chromatography A*, 1997, vol. 757, 303-306

- **C. VICENTE**. *Journal of Chromatography*, 1991, vol. 553, 271-283